# EUROPÄISCHE PATENTSCHRIFT

(11) **EP 1 361 840 B1**
(45) Veröffentlichungstag und Bekanntmachung des Hinweises auf die Patenterteilung: **28.09.2005**
(21) Anmeldenummer: 03743307.5
(22) Anmeldetag: 30.01.2003
(51) Int. Cl.: A61F 2/44, A61F 2/30, A61F 2/46

(54) **DISTRAHIERBARES WIRBELSÄULENIMPLANTAT**
DISTRACTIBLE VERTEBRAL COLUMN IMPLANT
IMPLANT VERTEBRAL CAPABLE DE DISTRACTION

(30) Priorität: 02.03.2002 DE 10210214
(43) Veröffentlichungstag der Anmeldung: 19.11.2003
(73) Patentinhaber: DePuy Spine Sàrl, 2400 Le Locle (CH)
(72) Erfinder: Schäfer, Bernd, 6315 Oberägeri (CH); Trautwein, Thilo, 70794 Filderstadt (DE); Liljenqvist, Ulf, 48147 Münster (DE)
(74) Vertreter: Steimle, Josef
(86) Internationale Anmeldenummer: PCT/EP2003/000932
(87) Internationale Veröffentlichungsnummer: WO 2003/073964

(56) Entgegenhaltungen:
- EP-A- 0 950 388
- WO-A-01/72246
- WO-A-98/46173

## Beschreibung

Die Erfindung betrifft ein distrahierbares Wirbelsäulenimplantat mit einer ersten Außenhülse und einer koaxial zu dieser angeordneten zweiten Außenhülse und einem inneren Antriebselement, welches teilweise mit wenigstens einer der Außenhülsen verschraubt ist, wobei das innere Antriebselement ein erstes Gewinde, z.B. ein Außengewinde und die mit dem Antriebselement verschraubte Außenhülse ein zweites, zum Außengewinde passendes Gewinde, z.B. ein Innengewinde aufweist.

Derartige Wirbelsäulenimplantate dienen als Zwischenwirbelimplantat für den Ersatz einzelner Wirbel, wie es z.B. aus der US 4,657,550 und EP 0 950 388 A2 bekannt ist. Bei diesen bekannten Zwischenwirbelimplantaten werden in jedes Ende einer Gewindehülse jeweils ein Gewindebolzen eingeschraubt, wobei die voneinander abgewandten Stirnseiten der Gewindebolzen in Stützplatten, die an den abzustützenden Wirbeln anliegen, eingreifen. Wird nun die Gewindehülse mittels eines radial einzusteckenden Stiftes oder mittels eines Hakenschlüssels gedreht, dann werden die beiden, ein Rechts- und ein Linksgewinde aufweisende Gewindestifte aus der Gewindehülse ausgeschraubt bzw. in diese eingeschraubt. Von Nachteil ist, dass für die Drehung der Gewindehülse das Werkzeug nach einer gewissen Umdrehung, z.B. nach einer Viertelumdrehung, umgesteckt werden muss, was bei einer Vielzahl von Operationen schwierig oder gar unmöglich ist.

Der Erfindung liegt daher die Aufgabe zugrunde, ein Wirbelsäulenimplantat, insbesondere ein Zwischenwirbelimplantat bereitzustellen, welches universeller einsetzbar und bei der Implantation wesentlich einfacher handhabbar ist.

Diese Aufgabe wird erfindungsgemäß mit einem distrahierbaren Wirbelsäulenimplantat gelöst, bei dem das innere Antriebselement auf einem Stützring aufliegt, und das Antriebselement im Bereich seiner dem Stützring zugewandten Stirnseite antreibbar ist.

Mit Vorzug weist das innere Antriebselement zwei Innenhülsen auf. Gemäß einer Weiterbildung ist jeweils eine Innenhülse in eine Außenhülse eingeschraubt. Dabei liegen vorzugsweise die Innenhülsen mit ihren einander zugewandten Stirnseiten auf einem Stützring auf, der diese insbesondere voneinander beabstandet.

Beim erfindungsgemäßen Wirbelsäulenimplantat gemäß Anspruch 1 werden die beiden äußeren Teile von einer Außenhülse gebildet, was den Vorteil hat, dass sie das Wirbelsäulenimplantat über eine größere Stützfläche am zugewandten Wirbel abstützt als beim oben genannten Stand der Technik. Es sind also keine Abstützplatten erforderlich. Außerdem wird durch den stirnseitigen Antrieb, insbesondere Kegelantrieb, vermieden, dass das Werkzeug nach einer gewissen Umdrehung immer neu angesetzt werden muss, da das Werkzeug nicht, wie beim Stand der Technik, am Umfang sondern an der Stirnseite des Antriebselements angreift.

Vorzugsweise wird das zwischen den beiden Außenhülsen sich befindende Antriebselement von zwei Innenhülsen gebildet, welche in die beiden Außenhülsen eingeschraubt sind. Dabei stützen sich die beiden einander zugewandten Stirnseiten der Innenhülsen an einen Stützring ab und werden von diesem beabstandet. Die beiden Innenhülsen sind im Bereich ihrer Stirnseite antreibbar, so dass sie in die Außenhülse eingeschraubt bzw. aus dieser ausgeschraubt werden können. Da die beiden Innenhülsen voneinander beabstandet sind, sind die beiden Stirnseiten über das Werkzeug zugänglich und können in Drehung versetzt werden, ohne dass ein Werkzeug ständig umgesetzt werden muss. Dies ist z.B. beim eingangs genannten Stand der Technik gemäß der US 4,657,550 der Fall, bei dem das mittlere Element mittels eines Stiftes oder Hakenschlüssels gedreht werden muss.

Bei einer Weiterbildung ist vorgesehen, dass die Stirnseite der Innenhülse mit einer Verzahnung, insbesondere einem Teil einer Kegelverzahnung versehen ist. Über diese Verzahnung kann mittels eines geeigneten Werkzeugs, z.B. dem anderen Teil der Kegelverzahnung die Innenhülse bzw. können die Innenhülsen in Drehbewegung versetzt werden, um die Schraubbewegung durchzuführen.

Bevorzugt weist der Stützring wenigstens eine radiale Öffnung auf. Die Öffnung dient dazu, um das Werkzeug zum einen in den Stützring hinein und an die Stirnseite der Innenhülse heranzuführen, und andererseits, um das Werkzeug zu führen und abzustützen. Ein geführtes und abgestütztes Werkzeug stellt eine weitaus geringere Gefahr bei Operationen dar, als nicht geführte und nicht abgestützte Werkzeuge.

Um die Innenhülsen aufzunehmen und zu lagern, weist der Stützring erfindungsgemäß eine radial nach innen vorspringende Schulter auf, die als Auflage für die Stirnseite der Innenhülse dient. Diese Schulter muss nicht an der Innenseite des Stützrings umlaufend vorgesehen sein, es ist ausreichend, wenn genügend Abschnitte vorgesehen sind, die die Hülse kippfrei lagern.

Bei einer Weiterbildung ist vorgesehen, dass die Öffnung und die Schulter sich schneiden. Wird durch die Öffnung ein Werkzeug in den Stützring eingeführt, dann befindet sich dieses im Bereich der Schulter und somit im Bereich der Abstützung der Innenhülse, so dass ein direkter Zugriff auf die Stirnseite, d.h. auf die Verzahnung der Innenhülse erfolgen kann.

Bei einer bevorzugten Variante ist vorgesehen, dass der Durchmesser der Öffnung größer ist als die Dicke der Schulter. Wird durch eine derartige Öffnung ein Werkzeug eingeschoben, dann steht dieses über die Schulter über und kann direkt an der Stirnseite der Hülse angreifen. Ist die Hülse, wie bei einer bevorzugten Weiterbildung der Erfindung vorgesehen, mit einer Verzahnung versehen, dann greift der über die Schulter überstehende Teil des Werkzeugs direkt in die Verzahnung ein, die in den lichten Querschnitt der Öffnung hineinragt.

Dabei kann die Verzahnung, wie bereits erwähnt, nach Art eines Kegelrades ausgeführt sein, so dass mit einem entsprechenden Werkzeug ein Zahnradgetriebe erzeugt werden kann. Dies hat den wesentlichen Vorteil, dass sehr feinfühlig und ohne Umsetzung des Werkzeugs die Innenhülse in der Außenhülse gedreht werden kann, wobei beliebige Drehstellungen möglich sind. Die Innenhülse muss also nicht bis in eine bestimmte Position verdreht werden, um z.B. das Werkzeug abnehmen bzw. neu ansetzen zu können.

Mit Vorzug weisen die Innenhülsen und die Außenhülsen jeweils ein Rechtsgewinde oder jeweils ein Linksgewinde auf. Dies hat den.wesentlichen Vorteil, dass für die beiden Innenhülsen gleiche Bauteile verwendet werden können, was auch für die Außenhülsen zutrifft, falls keine spezielle Anpassung an die Stellung oder Form der Wirbel erforderlich ist. Außerdem bedarf es lediglich eines Werkzeuges, bzw. einer Maschineneinstellung zum Herstellen des Innengewindes an der Außenhülse bzw. zum Herstellen des Außengewindes an der Innenhülse.

Um schnell eine optimale Verbindung des Implantats mit dem umgebenden Gewebe zu erzielen, weist die Mantelfläche der Innenhülsen und/oder der Außenhülsen Durchbrüche auf, welche leicht vom Knochengewebe durchwachsen werden können. Außerdem wird durch die Durchbrüche das Gesamtgewicht des Implantats verringert.

Bei einer Weiterbildung ist vorgesehen, dass wenigstens einer der Durchbrüche wenigstens einer Hülse eine Größe aufweist, um die Hülse mit Gewebematerial zu füllen bzw. zu ergänzen. Nach der Distraktion des erfindungsgemäßen Wirbelsäulenimplantats muss dieses mit zusätzlichem Gewebematerial aufgefüllt werden, welches über größere Durchbrüche leicht eingeführt werden kann. Diese größeren Durchbrüche befinden sich ebenfalls im Mantel wenigstens einer der Hülsen, wobei der Durchbruch bevorzugterweise eine längliche bzw. eine langovale Form besitzt.

Um eine optimale Anpassung des Wirbelsäulenimplantats an die Krümmung der Wirbelsäule zu erreichen, weist wenigstens eine der Außenhülsen eine nach außen weisende Stirnseite auf, die unter einem Winkel zur Orthogonalfläche bezüglich der Längsachse steht. Diese Außenfläche steht also nicht senkrecht zur Längsachse des Wirbelsäulenimplantats sondern ist zu dieser Ebene geneigt. Dabei stehen gemäß einem Baukastensystem verschiedene Außenhülsen zur Auswahl, die Außenflächen besitzen, die verschieden stark oder nicht geneigt sind.

Mit Vorzug sind diese Außenflächen mit dornartigen Fortsätzen versehen, so dass ein optimaler Halt am anliegenden Wirbel gewährleistet ist.

Um die endgültige Distraktionslage zu fixieren, sind die Außenhülse und die zugeordnete Innenhülse mittels einer radial in die Außenhülse einschraubbaren Madenschraube zueinander fixierbar. Dadurch ist gewährleistet, dass keine selbsttätige Verstellung, insbesondere Kontraktion des Wirbelsäulenimplantats erfolgt.

Um die beiden Innenhülsen miteinander zu koppeln, ist ein Rastelement vorgesehen, welches die beiden Innenhülsen am Stützring hält, wobei das Rastelement nach außen abragende Rastnasen, insbesondere an federnden Laschen, aufweist, die am Innenhülse vorgesehene, nach innen vorspringende Schultern hintergreifen. Ein weiterer Vorteil dieses Rastelements ist, dass das gesamte Wirbelsäulenimplantat modulartig aufbaubar ist und unmittelbar vor der Implantation zusammengesetzt werden kann. Dabei bedarf es keiner Werkzeuge, da das Rastelement lediglich in eine Innenhülse eingeschoben werden muss und die Schultern der benachbarten Innenhülse hintergreifen muss.

Weitere Vorteile, Merkmale und Einzelheiten der Erfindung ergeben sich aus der nachfolgenden Beschreibung, in der unter Bezugnahme auf die Zeichnung ein besonders bevorzugtes Ausführungsbeispiel der Erfindung im Einzelnen beschrieben ist. Dabei können die in der Zeichnung dargestellten sowie in den Ansprüchen und in der Beschreibung erwähnten Merkmale jeweils einzeln für sich oder in beliebiger Kombination erfindungswesentlich sein.

In der Zeichnung zeigen:
- Fig. 1:: eine perspektivische Ansicht eines Ausführungsbeispiels des erfindungsgemäßen distrahierbaren Wirbelsäulenimplantats;
- Fig. 2:: das Wirbelsäulenimplantat gemäß Fig. 1 in distrahierter Stellung;
- Fig. 3:: das Wirbelsäulenimplantat gemäß Fig. 1 in Expositionsdarstellung;
- Fig. 4:: eine vergrößerte Wiedergabe einer perspektivischen Darstellung der oberen Außenhülse gemäß Fig. 3;
- Fig. 5:: eine vergrößerte Wiedergabe einer perspektivischen Darstellung der unteren Außenhülse gemäß Fig. 3;
- Fig. 6:: eine vergrößerte Wiedergabe einer perspektivischen Darstellung einer Innenhülse;
- Fig. 7:: einen Längsschnitt durch die Innenhülse;
- Fig. 8:: eine vergrößerte Wiedergabe einer perspektivischen Darstellung eines Stützringes;
- Fig. 9:: eine vergrößerte Wiedergabe einer perspektivischen Darstellung eines Rastelements, und
- Fig. 10: eine perspektivische Darstellung eines Werkzeugs zum Distrahieren des Wirbelsäulenimplantats.

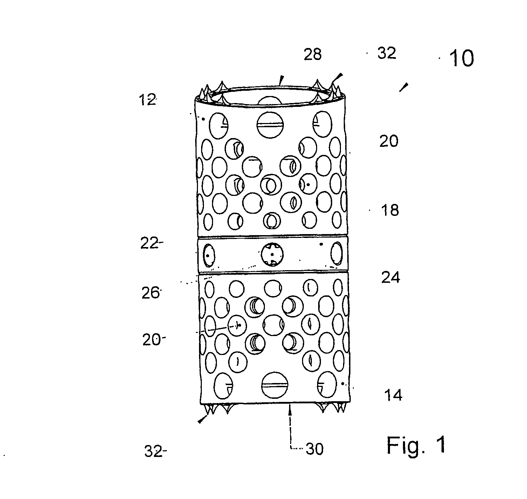

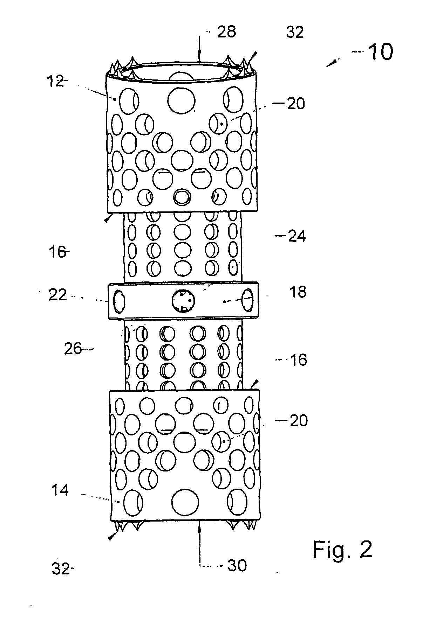

Die Fig. 1 zeigt ein bevorzugtes Ausführungsbeispiel eines insgesamt mit 10 bezeichneten Wirbelsäulenimplantats, welches seine komprimierte Lage einnimmt. Erkennbar sind eine obere Außenhülse 12 sowie eine untere Außenhülse 14, die mit ihren einander zugewandten Stirnseiten 16 (siehe Fig. 2) an einem Stützring 18 anliegen. Außerdem ist erkennbar, dass die Außenhülsen 12 und 14 mit Durchbrüchen 20 versehen sind, durch welche Knochengewebe in das Innere des Wirbelsäulenimplantats 10 einwachsen kann.

Der Stützring 18 ist mit radial verlaufenden Öffnungen 22 versehen, durch deren lichten Querschnitt 24 Teile einer Verzahnungen 26 erkennbar sind. Schließlich weisen die beiden Außenhülsen 12 und 14 nach außen hin gerichtete Stirnseiten 28 und 30 auf, die ihrerseits mit dornartigen Fortsätzen 32, welche axial abragen, versehen sind. Diese dornartigen Fortsätze 32 dringen in die Anlageflächen der benachbarten Wirbel ein und verankern dort die beiden Außenhülsen 12 und 14.

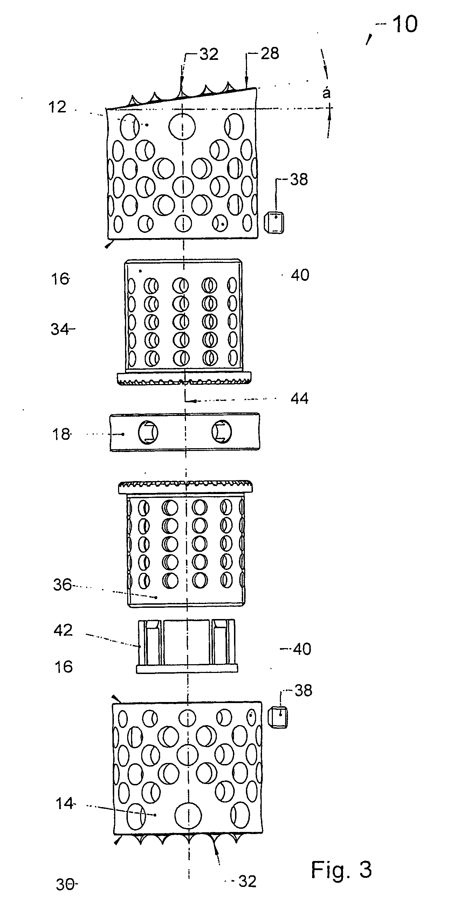

In den Fig. 2 und 3 sind eine obere Innenhülse 34 und eine untere Innenhülse 36 dargestellt, die ihrerseits in die zugehörige obere Außenhülse 12 bzw. untere Außenhülse 14 eingeschraubt sind. Weiterhin sind zwei Madenschrauben 38 dargestellt, die in eine entsprechende Gewindebohrung 40 der Außenhülse 12 bzw. 14 eingeschraubt werden kann, wodurch die Außenhülse 12 bzw. 14 an der Innenhülse 34 bzw. 36 fixiert werden kann. Die Gewindebohrungen 40 für die Madenschrauben 38 befinden sich in unmittelbarer Nachbarschaft zu den Stirnseiten 16 der Außenhülsen 12 und 14. Schließlich ist noch ein Rastelement 42 erkennbar, mit welchem die beiden Innenhülsen 34 und 36 aneinander befestigt werden.

Deutlich erkennbar ist, dass die Ebene der Stirnseite 28 gegenüber der Orthogonalebene zur Längsachse 44 um einen Winkel α geneigt ist. Dadurch kann das Wirbelsäulenimplantat 10 optimal an die Stellung der benachbarten Wirbel angepasst, bzw. kann deren Stellung korrigiert werden. Dabei wird jeweils eine Außenhülse 12 bzw. 14 ausgewählt, die eine Stirnseite 28 bzw. 30 mit der erforderlichen Neigung aufweist. Weiterhin ist erkennbar, dass die Hülsen 12, 14 34 und 36 sowie der Stützring 18 und das Rastelement 42 koaxial zueinander und bezüglich der Längsachse 44 angeordnet sind.

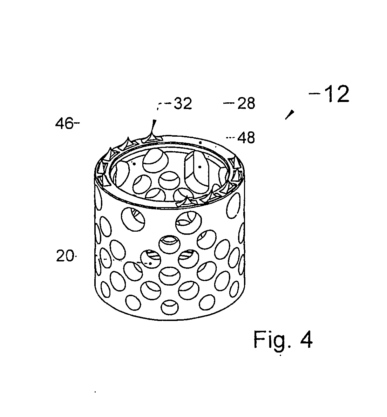

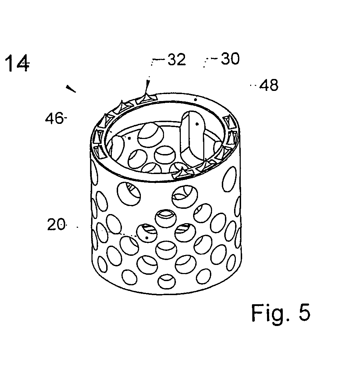

In den Fig. 4 und 5 sind die beiden Außenhülsen 12 bzw. 14 in vergrößerter Darstellung wiedergegeben, wobei jedoch das am Innenumfang vorgesehene Innengewinde 46 lediglich schematisch dargestellt bzw. angedeutet ist. Dieses Innengewinde 46 ist z.B. ein Feingewinde mit einer Steigung von 1 mm und einem Durchmesser von 22 mm und als Rechtsgewinde ausgebildet.

Außerdem ist in den Fig. 4 und 5 erkennbar, dass ein größerer, länglicher Durchbruch 48 in der Wandung der Außenhülsen 12 und 14 vorgesehen ist, durch welchen nach der Distraktion Knochengewebe ins Innere des Wirbelsäulenimplantats 10 eingefüllt werden kann.

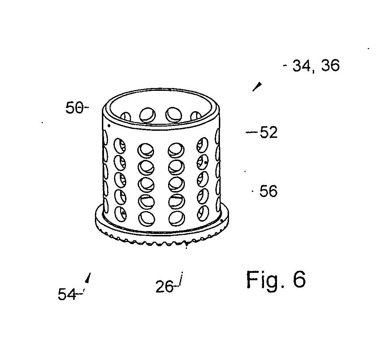

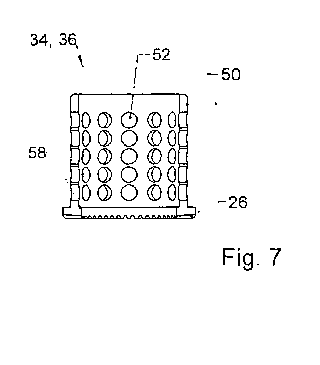

Die Fig. 6 zeigt eine vergrößerte perspektivische Darstellung der Innenhülse 34 bzw. 36, die an ihrem Außenumfang mit einem Außengewinde 50 versehen ist, welches ebenfalls nur schematisch dargestellt bzw. angedeutet ist. Auch die Innenhülse 34 bzw. 36 ist mit Durchbrüchen 52 versehen. Die Innenhülse 34 bzw. 36 besitzt an einer ihrer Stirnseiten 54 einen radial nach außen abragenden Flansch 56, der an seiner nach außen gewandten Stirnseite mit der Verzahnung 26, die insbesondere als Kegelverzahnung ausgebildet ist, versehen ist. Im in der Fig. 7 dargestellten Längsschnitt ist diese Kegelverzahnung deutlich erkennbar.

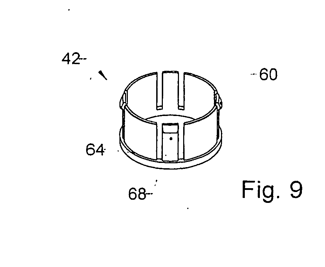

Außerdem ist erkennbar, dass der Flansch 56 eine radial nach innen vorspringende Schulter 58 aufweist, an welcher Rastnasen 60 eines Rastelements 42 verrasten können. Ein derartiges Rastelement 42 ist in Fig. 9 dargestellt. Dieses Rastelement 42 ist ebenfalls hülsenförmig ausgebildet und besitzt an seiner den Rastnasen 60 gegenüber liegenden Seite einen radial vorspringenden Halteflansch 64, der hinter der entsprechenden Schulter 58 der anderen Innenhülse 36 zu liegen kommt. Die Rastnasen sind an federnden Laschen 68 vorgesehen, so dass sie radial nach innen ausgelenkt werden können. Auf diese Weise können die beiden Innenhülsen 34 und 36 miteinander verbunden werden.

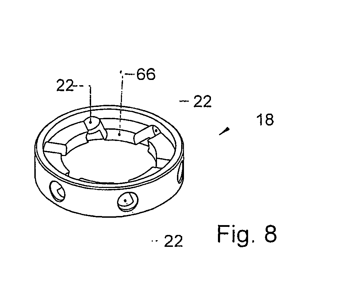

Die Fig. 8 zeigt den Stützring 18 auf dem die beiden Innenhülsen 34 und 36 mit ihren Verzahnungen 26 aufsitzen. Hierfür weise der Stützring 18 eine radial nach innen vorspringende Schulter 66 auf, die in insgesamt sechs Segmente unterteilt ist. Die Schulter 66 ist so angeordnet, dass sie die Öffnungen 22 schneidet, wobei der Durchmesser der Öffnungen 22 größer ist, als die Dicke der Schulter 66. Dies hat zur Folge, dass ein Teil der Verzahnung 26 in den lichten Querschnitt der Öffnungen 22 hineinragt, wenn die Innenhülsen 34 und 36 auf der Schulter 66 aufsitzen. Auf die Verzahnung 26 kann dann durch die Öffnung 22 hindurch von außen zugegriffen werden, wie es in den Fig. 1 und 2 gezeigt ist.

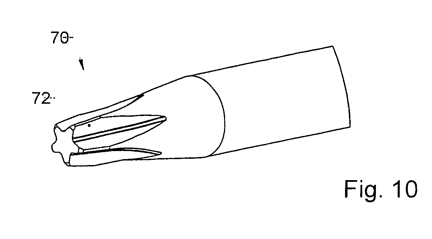

Schließlich ist in der Fig. 10 noch ein Werkzeug 70 dargestellt, welches eine längliche Gestalt besitzt und im Querschnitt sternförmig ausgebildet ist. Das Werkzeug 70 besitzt ebenfalls eine Verzahnung 72, die zusammen mit der Verzahnung 26 ein Kegelgetriebe bilden. Das Werkzeug kann an einem starren Stab oder an einer biegsamen Welle vorgesehen sein, so dass auch schwierig zugängliche Stellen problemlos mit dem Werkzeug 70 erreichbar und das Wirbelsäulenimplantat 10 distrahierbar ist.

## Patentansprüche

1. Distrahierbares Wirbelsäulenimplantat (10) mit einer ersten Außenhülse (12) und einer koaxial zu dieser angeordneten zweiten Außenhülse (14) und einem inneren Antriebselement, welches mit wenigstens einer der Außenhülsen (12, 14) verschraubt ist, wobei das innere Antriebselement ein erstes Gewinde, z.B. ein Außengewinde (50) und die mit dem Antriebselement verschraubte Außenhülse (12, 14) ein zweites, zum Außengewinde (50) passendes Gewinde, z.B. ein Innengewinde (46) aufweist, **dadurch gekennzeichnet, dass** das innere Antriebselement auf einem Stützring (18) aufliegt, und das Antriebselement im Bereich seiner dem Stützring (18) zugewandten Stirnseite (54) antreibbar ist.

2. Wirbelsäulenimplantat nach einem der vorhergehenden Ansprüche, **dadurch gekennzeichnet, dass** das Antriebselement zwei Innenhülsen (34 und 36) aufweist.

3. Wirbelsäulenimplantat nach Anspruch 2, **dadurch gekennzeichnet, dass** jeweils eine Innenhülse (34 bzw. 36) in eine Außenhülse (12 und 14) eingeschraubt ist.

4. Wirbelsäulenimplantat nach Anspruch 2 oder 3, **dadurch gekennzeichnet, dass** die Innenhülsen (34 und 36) über den Stützring (18) voneinander beabstandet sind.

5. Wirbelsäulenimplantat nach einem der vorhergehenden Ansprüche, **dadurch gekennzeichnet, dass** die Stirnseite (54) des Antriebselements oder der Innenhülse (34 bzw. 36) mit einer Verzahnung (26) versehen ist.

6. Wirbelsäulenimplantat nach einem der vorhergehenden Ansprüche, **dadurch gekennzeichnet, dass** der Stützring (18) wenigstens eine radiale Öffnung (22) aufweist.

7. Wirbelsäulenimplantat nach einem der vorhergehenden Ansprüche, **dadurch gekennzeichnet, dass** der Stützring (18) eine radial nach innen vorspringende Schulter (66) aufweist, die als Auflage für die Stirnseite (54) der Innenhülse (34, 36) dient.

8. Wirbelsäulenimplantat nach Anspruch 6 und 7, **dadurch gekennzeichnet, dass** die Öffnung (22) und die Schulter (66) sich schneiden.

9. Wirbelsäulenimplantat nach Anspruch 8, **dadurch gekennzeichnet, dass** der Durchmesser der Öffnung (22) größer ist als die Dicke der Schulter (66).

10. Wirbelsäulenimplantat nach Anspruch 5 und 6, **dadurch gekennzeichnet, dass** die Verzahnung (26) in den lichten Querschnitt der Öffnung (22) hineinragt.

11. Wirbelsäulenimplantat nach einem der vorhergehenden Ansprüche, **dadurch gekennzeichnet, dass** die Innenhülsen (34 und 36) und die Außenhülsen (12 und 14) jeweils ein Rechtsgewinde oder jeweils ein Linksgewinde aufweisen.

12. Wirbelsäulenimplantat nach einem der vorhergehenden Ansprüche, **dadurch gekennzeichnet, dass** die Mantelflächen der Innenhülsen (34 und 36) und/oder der Außenhülsen (12 und 14) mit Durchbrüchen (20, 48) versehen sind.

13. Wirbelsäulenimplantat nach Anspruch 12, **dadurch gekennzeichnet, dass** wenigstens einer der Durchbrüche (48) wenigstens einer Hülse (12, 14, 34 oder 36) eine Größe aufweist, so dass die Hülse (12, 14, 34 oder 36) mit Gewebematerial füllbar ist.

14. Wirbelsäulenimplantat nach einem der vorhergehenden Ansprüche, **dadurch gekennzeichnet, dass** eine der Außenhülsen (12 oder 14) eine nach außen weisende Stirnseite (28) aufweist, die unter einem Winkel (α) zur Orthogonalfläche bezüglich der Längsachse (44) steht.

15. Wirbelsäulenimplantat nach einem der vorhergehenden Ansprüche, **dadurch gekennzeichnet, dass** die Außenhülse (12, 14) und die zugeordnete Innenhülse (34, 36) mittels einer radial in die Außenhülse (12, 14) einschraubbaren Madenschraube (38) aneinander fixierbar sind.

16. Wirbelsäulenimplantat nach einem der vorhergehenden Ansprüche, **dadurch gekennzeichnet, dass** die beiden Innenhülsen (34 und 36) durch ein Rastelement (42) am Stützring (18) gehalten werden, wobei das Rastelement (42) nach außen abragende Rastnasen (60) aufweist, die an der Innenhülse (34, 36) vorgesehene, nach innen vorspringende Schultern (58) hintergreifen.

## Claims

1. An extendable spinal implant (10) with a first outer sleeve (12) and a second outer sleeve (14) coaxially disposed thereto and an inside drive element which is screwed to at least one of the outer sleeve (12, 14), with said inside drive element having a first thread, e.g., an outside thread (50), and with the outer sleeve (12, 14) that is connected by means of screws to the drive element having a second thread, e.g., an inside thread (46), that fits on the outside thread (50), **characterized in that** the inside drive element is seated on a supporting ring (18) and that the drive element can be driven in the area of its front surface (54) that faces the supporting ring (18).

2. The spinal implant as claimed in one of the preceding claims, **characterized in that** the drive element comprises two inner sleeves (34 and 36).

3. The spinal implant as claimed in claim 2, **characterized in that** each inner sleeve (34 and 36) is screwed into an outer sleeve (12 and 14).

4. The spinal implant as claimed in claim 2 or 3, **characterized in that** the inner sleeves (34 and 36) are spaced a certain distance apart by means of the supporting ring (18).

5. The spinal implant as claimed in any one of the preceding claims, **characterized in that** the front surface (54) of the drive element or the inner sleeve (34 and 36) has teeth (26).

6. The spinal implant as claimed in any one of the preceding claims, **characterized in that** the supporting ring (18) has at least one radial opening (22).

7. The spinal implant as claimed in any one of the preceding claims, **characterized in that** the supporting ring (18) has a shoulder (66) which projects radially inwardly and which serves as a support for the front surface (54) of the inner sleeve (34, 36).

8. The spinal implant as claimed in Claims 6 and 7, **characterized in that** the opening (22) and the shoulder (66) intersect with each other.

9. The spinal implant as claimed in Claim 8, **characterized in that** the diameter of the opening (22) is greater than the thickness of the shoulder (66).

10. The spinal implant as claimed in Claims 5 and 6, **characterized in that** the teeth (26) project into the inside cross-section of the opening (22).

11. The spinal implant as claimed in any one of the preceding claims, **characterized in that** each of the inner sleeves (34 and 36) and the outer sleeves (12 and 14) have a right-hand thread or a left-hand thread.

12. The spinal implant as claimed in any one of the preceding claims, **characterized in that** the surfaces of the inner sleeves (34 and 36) and/or the outer sleeves (12 and 14) have perforations (20,48).

13. The spinal implant as claimed in Claim 12, **characterized in that** at least one of the perforations (48) of at least one sleeve (12, 14, 34 or 36) is large enough so as to make it possible to fill the sleeve (12, 14, 34 or 36) with tissue substance.

14. The spinal implant as claimed in any one of the preceding claims, **characterized in that** one of the outer sleeves (12 or 14) has an outwardly projecting front surface (28) which is positioned at an angle (α) to the orthogonal plane relative to the longitudinal axis (44).

15. The spinal implant as claimed in any of the preceding claims, **characterized in that** the outer sleeve (12,14) and the associated inner sleeve (34, 36) can be affixed to each other by means of a grub screw (38) that can be radially screwed into the outer sleeve (12, 14).

16. The spinal implant as claimed in any one of the preceding claims, **characterized in that** the two inner sleeves (34 and 36) are maintained on the supporting ring (18) by means of a locking element (42), with said locking element (42) having outwardly projecting detents (60) which extend behind inwardly projecting shoulders (58) that are disposed on the inner sleeve (34, 36).

## Revendications

1. Implant vertébral capable de distraction (10), comportant une première gaine extérieure (12) et une deuxième gaine extérieure (14) disposée de manière coaxiale par rapport à celle-ci, et un élément d'entraînement intérieur, qui est vissé avec au moins l'une des gaines extérieures (12, 14), moyennant quoi l'élément d'entraînement intérieur présente un premier filetage, par exemple un filetage extérieur (50), et la gaine extérieure (12, 14) vissée avec l'élément d'entraînement présente un deuxième filetage adapté au filetage extérieur (50), par exemple un filetage intérieur (46), **caractérisé en ce que** l'élément d'entraînement intérieur repose sur une bague d'appui (18), et l'élément d'entraînement peut être entraîné dans la zone de son côté frontal (54) orienté vers la bague d'appui (18).

2. Implant vertébral selon l'une quelconque des revendications précédentes, **caractérisé en ce que** l'élément d'entraînement présente deux gaines intérieures (34 et 36).

3. Implant vertébral selon la revendication 2, **caractérisé en ce que** respectivement une gaine intérieure (34 ou 36) est intégrée par vissage dans une gaine extérieure (12 et 14).

4. Implant vertébral selon l'une quelconque des revendications 2 ou 3, **caractérisé en ce que** les gaines intérieures (34 et 36) sont espacées l'une de l'autre par l'intermédiaire de la bague d'appui (18).

5. Implant vertébral selon l'une quelconque des revendications précédentes, **caractérisé en ce que** le côté frontal (54) de l'élément d'entraînement ou de la gaine intérieure (34 ou 36) est doté d'une denture (26).

6. Implant vertébral selon l'une quelconque des revendications précédentes, **caractérisé en ce que** la bague d'appui (18) présente au moins une ouverture radiale (22).

7. Implant vertébral selon l'une quelconque des revendications précédentes, **caractérisé en ce que** la bague d'appui (18) présente un épaulement (66) faisant saillie radialement vers l'intérieur, qui sert de support pour le côté frontal (54) de la gaine intérieure (34, 36).

8. Implant vertébral selon l'une quelconque des revendications 6 et 7, **caractérisé en ce que** l'ouverture (22) et l'épaulement (66) se coupent.

9. Implant selon la revendication 8, **caractérisé en ce que** le diamètre de l'ouverture (22) est supérieur à l'épaisseur de l'épaulement (66).

10. Implant vertébral selon l'une quelconque des revendications 5 et 6, **caractérisé en ce que** la denture (26) mord dans la section libre de l'ouverture (22).

11. Implant vertébral selon l'une quelconque des revendications précédentes, **caractérisé en ce que** les gaines intérieures (34 et 36) et les gaines extérieures (12 et 14) présentent respectivement un filetage à droite ou respectivement un filetage à gauche.

12. Implant vertébral selon l'une quelconque des revendications précédentes, **caractérisé en ce que** les surfaces d'enveloppe des gaines intérieures (34 et 36) et / ou des gaines extérieures (12 et 14) sont dotées d'ajours (20, 48).

13. Implant vertébral selon la revendication 12, **caractérisé en ce qu'**au moins l'un des ajours (48) d'au moins une gaine (12, 14, 34 ou 36) a une taille telle que la gaine (12, 14, 34 ou 36) peut être remplie de matériau tissé.

14. Implant vertébral selon l'une quelconque des revendications précédentes, **caractérisé en ce que** l'une des gaines extérieures (12 ou 14) présente un côté frontal (28) orienté vers l'extérieur, qui représente un angle (α) avec la surface orthogonale par rapport à l'axe longitudinal (44).

15. Implant vertébral selon l'une quelconque des revendications précédentes, **caractérisé en ce que** la gaine extérieure (12, 14) et la gaine intérieure (34, 36) associée peuvent être fixées l'une à l'autre à l'aide d'une vis sans tête (38) pouvant être insérée radialement dans la gaine extérieure (12, 14).

16. Implant vertébral selon l'une quelconque des revendications précédentes, **caractérisé en ce que** les deux gaines intérieures (34 et 36) sont maintenues sur la bague d'appui (18) grâce à un élément d'enclenchement (42), moyennant quoi l'élément d'enclenchement (42) présente des taquets (60) dépassant vers l'extérieur, qui mordent dans les épaulements (58) faisant saillie vers l'intérieur, prévus sur la gaine intérieure (34, 36).
